# EUROPÄISCHE PATENTSCHRIFT

(11) **EP 1 819 729 B1**
(45) Veröffentlichungstag und Bekanntmachung des Hinweises auf die Patenterteilung: **22.04.2009**
(21) Anmeldenummer: 05850211.3
(22) Anmeldetag: 18.11.2005
(51) Int. Cl.: C07K 14/605, C07K 14/575, A61K 38/22, A61K 38/26, C12N 15/16

(54) **VERFAHREN ZUR HERSTELLUNG VON CARBOXY-TERMINAL AMIDIERTEN PEPTIDEN**
METHOD FOR PRODUCING CARBOXY-TERMINAL AMIDIFIED PEPTIDES
PROCEDE DE PRODUCTION DE PEPTIDES A AMIDIFICATION CARBOXY-TERMINALE

(30) Priorität: 01.12.2004 DE 102004058306
(43) Veröffentlichungstag der Anmeldung: 22.08.2007
(73) Patentinhaber: Sanofi-Aventis Deutschland GmbH, 65929 Frankfurt am Main (DE)
(72) Erfinder: HABERMANN, Paul, 65817 Eppstein (DE); DECKER, Heinrich, 65926 Frankfurt (DE); LATTEMANN, Claus, 65812 Bad Soden (DE); MANEG, Oliver, 65936 Frankfurt (DE); SALAGNAD, Christophe, 61440 Oberursel (DE); ZOCHER, Frank, 65926 Frankfurt (DE)
(86) Internationale Anmeldenummer: PCT/EP2005/012365
(87) Internationale Veröffentlichungsnummer: WO 2006/058620

(56) Entgegenhaltungen:
- EP-A- 1 076 066
- WO-A-00/28067
- WO-A-96/04373
- WO-A-96/17941
- US-A1- 2004 106 547
- ROURKE IJ ET AL.: "Heterologous Expression of Human Cholecystokinin in Saccharomyces cerevisiae" THE JOURNAL OF BIOLOGICAL CHEMISTRY, Bd. 272, Nr. 15, 11. April 1997 (1997-04-11), Seiten 9720-9727, XP002383083
- ISHIKAWA H & TAMAOKI H: "Production of Human Calcitonin in Escherichia coli from Multimeric Fusion Protein" JOURNAL OF FERMENTATION AND BIOENGINEERING, Bd. 82, Nr. 2, 1996, Seiten 140-144, XP002383084

## Beschreibung

Die vorliegende Erfindung betrifft die Herstellung von Carboxy-terminal (C-terminal) amidierten Peptiden, mit C-terminal amidiertem Lysin, insbesondere mit der biologischen Aktivität von GLP-1, deren chemische bzw. biotechnologische Vorstufen und Zwischenprodukte, Verfahren zu deren Herstellung sowie deren Verwendungen zur Herstellung pharmazeutischer Produkte.

Die Zahl der an Diabetes oder Obesitas erkrankten Menschen steigt weltweit mit hohen Zuwachsraten an. Es ist daher zu erwarten, dass Medikamente, die im Feld dieser Erkrankung hohen therapeutischen Nutzen zeigen, in steigender Qualität und Menge zugänglich gemacht werden müssen.

Patentanmeldung US2004/0106547 A1 beschreibt von Exendin abgeleitete Peptide, die aufgrund ihrer Blutzucker-senkenden Wirkung in der Behandlung von Diabetes oder anderen Stoffwechselstörungen, die z.B. zur Fettleibigkeit führen können, als mögliche Medikamente eine wichtige Rolle spielen. Aufgrund ihres physiologischen Wirkungsmechanismus vermutet man derzeit insbesondere, dass sich diabetische Spätfolgen weniger ausgeprägt bzw. stark verzögert einstellen.

Die in US2004/0106547A1 beschriebenen Peptide erweisen sich aufgrund der Einführung von einem oder mehreren C-terminalen Lysinresten, von denen das endständige C-terminal amidiert ist, als besonders wirksam.

Für die Herstellung dieser Peptide werden in US2004/0106547A1 verschiedene Herstellverfahren genannt. Eines betrifft ein biotechnologisches Verfahren, worin nach intrazellulärer Expression in Hefen die Isolation des Zielproteins aus dem Zellaufschluss erfolgt. Peptide, die C-terminal amidiert sind, werden von Mikroorganismen aber nur in Spuren gebildet, so dass eine biotechnologische Herstellung, wie sie US2004/0106547A1 vorschlägt, nur mit großem Aufwand bzw. kostenintensiv erfolgen kann.

Alternativ beschreibt die Anmeldung die chemische Vollsynthese der jeweiligen Peptide. Dabei wird eine modifizierte Merryheld-Synthese vorgeschlagen, welche aber immer noch sehr aufwendig und mit hohen Kosten verbunden ist. Die Gründe hierfür liegen u.a. darin, dass die zur Synthese verwendeten Aminosäuren zunächst hergestellt und gereinigt werden müssen, um anschließend nach chemischer Modifikation in der Peptidsynthese als Reaktanden spezifisch eingesetzt zu werden. Am Ende der Synthese müssen die Schutzgruppen entfernt werden und das Zielpeptid bzw. Produkt gereinigt werden, bevor es als Pharmazeutikum formuliert werden kann. Die chemische Vollsynthese ist also mit hohem und im ökologischen Sinn wenig vorteilhaftem Aufwand durchführbar.

Es sind bereits seit langem Enzyme bekannt, die in der Lage sind, Peptide C-terminal zu amidieren. Diese Enzyme tragen den Namen (Eipper et al. Mol. Endocrinol.1987 Nov;1 (11):1987) Peptidylglycine alpha-amidating Enzym (PAM). Die Herstellung und Reinigung solcher PAM-Enzyme ist dem Fachmann geläufig und im Detail beschrieben: Solche Enzympräparationen sind darüber hinaus vielfach kommerziell erhältlich (z.B. K.Ohsuye et al., Cytotechnology 31,1999: 85-94, US4708934, US5789234, US6255067, US6319685, JP0177184).

Bradbury et al. (Biochem. Biophys Res. Commun. (1983) 112(2):372-377 zeigten 'in vitro', dass PAM als Substrat vorzugsweise Peptide erkennt, deren C-Terminus durch die Aminosäure Glycin gebildet wird. Sie beschreiben ferner, dass basische Aminosäuren in N-terminaler Position zu Glycin die Reaktionsgeschwindigkeit des PAM stark verlangsamen.

Es wurde nun gefunden, dass Exendin-Derivate mit einer C-terminalen Sequenz basischer Aminosäuren, insbesondere einer oligo- bzw. poly-Lysin-Sequenz, die ferner einen C-terminalen Glycin-Rest tragen, überraschend gut von PAM als Substrat erkannt werden.

Somit wird erfindungsgemäß überraschenderweise eine wesentlich kostengünstigere biotechnologische Herstellung von amidierten Peptiden ermöglicht, insbesondere solcher amidierter Peptide, wie sie in US2004/0106547A1 beschrieben sind, wobei das gewünschte Produkt aus seinem C-terminal um Glycin verlängerten Vorläuferpeptid/protein mittels eines einzigen enzymatischen Schrittes darstellbar ist.

In dem erfindungsgemäßen Verfahren werden biologisch aktive Peptide dargestellt, die ein oder mehrere basische Aminosäuren, vorzugsweise Lysin-, Histidin- und/oder Arginin-Reste, insbesondere Lysin-Reste mit einem C-terminale Lysinreste enthalten, wobei der letzte C-terminale Lysinrest C-terminal amidiert ist. Vorzugsweise weisen die nach dem erfindungsgemäßen Verfahren hergestellten Peptide die biologische Aktivität von GLP-1, Exendin-4, oder deren biologisch aktiver Analoga oder Derivate auf.
Die vorliegende Erfindung erlaubt somit insbesondere die biotechnologische Darstellung der Verbindung Nr. 2 aus US2004/0106547. Die besagte Verbindung Nr. 2 hat die Sequenz (Seq ID No 1):
NH₂-HGEGTFTSDL SKQMEEEAVR LFIEWLKNGG PSSGAPPSKK KKKK-NH₂

Gegenstand der vorliegenden Erfindung sind

Verfahren zur Herstellung von C-terminal amidierten Peptiden der allgemeinen Formel II

(AS)n-Xm-NH₂ (Formel II),

worin
- AS: eine oder mehrere genetisch codierbare Aminosäure bedeutet;
- n: 5-2000 ist, vorzugsweise 10-1000, insbesondere 15-500, ganz besonders bevorzugt 20-400;
und
- (AS)n: und/oder (AS)n-Xm ein biologisch aktives Peptid bzw. Protein bedeutet,
- X: ein oder mehrere basische Aminosäure oder deren Derivate, vorzugsweise
- Lysin,: Histidin und/oder Arginin, insbesondere Lysin bedeutet;
- m: 1-15 ist, vorzugsweise 3-10, insbesondere 6-8; und
- n und m: ganze Zahlen bedeuten, und
worin man
a) die erfindungsgemäßen Wirtszellen in einem geeigneten Nährmedium kultiviert,
b) die erfindungsgemäßen Peptide exprimiert,
c) gegebenenfalls die erfindungsgemäßen Peptide aus einem geeigneten Vorläuferpeptid mittels enzymatischer Spaltung freisetzt;
d) die Expressionsprodukte aus Schritt b) bzw. die Zwischenprodukte aus Schritt c)-gegebenenfalls nach erfolgter Aufreinigung-mit einem alpha-amidierenden Enzym zu Verbindungen der allgemeinen Formel II umsetzt; und
e) die Verbindungen der allgemeinen Formel II in geeigneter Weise aufreinigt, vorzugsweise durch präparative chromatographische Verfahren.

Dabei ist dem Fachmann bewußt, dass auch die Kombinationen bekannter biochemischer bzw. biophysikalischer Trennmethoden zu dem gewünschten Reinigungserfolg führen können.

Das erfindungsgemäße Verfahren dient zur Herstellung von Verbindungen der Formel I nach Seq ID No 2: NH₂- HGEGTFTSDL SKQMEEEAVR LFIEWLKNGG PSSGAPPSKK KKKKG (Seq ID No 2) sowie deren biologisch aktive Derivate mit einer Homologie von mindestens 60%, vorzugsweise 80%, insbesondere 90%.

Vorzugsweise dient das erfindungsgemäße Verfahren zur Herstellung von C-terminal amidierten Peptiden der Herstellung von Verbindungen gemäß Seq ID No 1.

Zunächst wird im Rahmen des erfindungsgemäßen Verfahrens die Fähigkeit von Mikroorganismen ausgenutzt, heterologe Peptide/Proteine herzustellen. Dazu übersetzt man die gewünschte Peptid/Proteinsequenz in die entsprechende DNA-Sequenz, die an eine wirtsspezifische Promotorsequenz gekoppelt wird. Je nach Expressionsstrategie kann man dabei das Zielpeptid so exprimieren, dass es von den Zellen intrazellulär verbleibend direkt oder indirekt als Fusionsprotein gebildet wird. Das Fusionsprotein kann entweder direkt mit PAM umgesetzt werden, bevor es chemisch oder enzymatisch zu dem gewünschten Zielprotein prozessiert wird, oder es wird - in umgekehrter Abfolge - zuerst in die Fusionsteile gespalten, bevor die Amidierung durch Umsetzung mit PAM erfolgt. Wählt man eine Fusionsstrategie, dann ist dem Fachmann klar, dass die Fusionspartner über ein Brückenglied miteinander verbunden sein müssen, dass die Aufspaltung der Partner so erlaubt, dass der N-Terminus des um Lys-Xₘ-Gly verlängerten Zielpeptides korrekt nach der Prozessierung vorliegt. Für die Ausgestaltung des Brückengliedes gibt es eine Vielzahl von Möglichkeiten. Wählt man z.B. die Aminosäure Methionin, so wird eine chemische Spaltung mit Halogencyan möglich. Wählt man z.B. als Brückenglied ein Pentapeptid der Sequenz DDDDK so ist eine Spaltung mit Enterokinase möglich. Wählt man z.B. die Tetrapeptidsequenz IEGR, so kann die Abspaltung über Faktor Xa erfolgen. Bei entsprechender Ausgestaltung kann man für Proteine, deren N-Terminus mit Histidin beginnen, Genenase^{®} als Prozessierungsenzym verwenden. Im Beispielteil wird nachfolgend die Abspaltung mittels Enterokinase beschrieben.

Alternativ kann man aber auch das Zielpeptid, wenn es exportkompatibel ist, in das Medium entweder in Form eines Fusionsproteins oder direkt in nativer Form ausschleusen. Dafür kann man gentechnisch modifizierte Zellen, insbesondere von Mikroorganismen, vorzugsweise Bakterien oder Hefen, verwenden. Wählt man Bakterienzellen als Expressionssystem hat man ferner die Option, direkt das Zielprotein oder ein entsprechendes Fusionsprotein, welches das Zielprotein umfasst, in das Periplasma oder in das Kulturmedium auszuschleusen.

Dem Fachmann sind die hierfür grundsätzlich zur Verfügung stehenden Wirtsorganismen und Methoden bekannt. Diese sind zum großen Teil auch kommerziell von einer Vielzahl von Anbietern erhältlich. Stellvertretend seien die Firmen New England Biolabs, Invitrogen und Roche genannt. In den Katalogbeschreibungen solcher Firmen finden sich Literaturzitate, die einen Überblick über die Technologie liefern.

Dem Fachmann ist dabei auch klar, dass sich das Spektrum der zur Verwendung kommenden Mikroorganismen ständig erweitert, ebenso wie das biotechnologische Methodenrepertoire. Auch die in dieser Hinsicht spezialisierten Ausführungsformen werden vom Gegenstand der vorliegenden Erfindung umfasst.

Stellvertretend werden beispielhaft folgende Wirts/Vektorsystemen genannt: Bakterien des Typs E.coli , S.carnosus, Salmonella, Bacillus subtilis oder Pseudomonas sowie Hefen vom Typ K. lactis, P. pastoris, Schizosaccharomyces pombe und S. cerevisiae.

Nachfolgend ist beispielhaft die Verwendung von Systemen basierend auf E.coli K12 bzw. E. coli B beschrieben. Dem Fachmann ist jedoch bewusst, dass diese beispielhaft genannten Systeme eine Vielzahl von Variationsmöglichkeiten bieten, die sich z.B. aus der Wahl geeigneter Promotoren oder anderer regulatorischer Nukleinsäuresequenzen, den genetischen Eigenschaften der Wirtszelle und der verwendeten Vektoren ergeben (z.B. Kopienzahl der DNA, Selektionsmittel etc.). Es ist dem Fachmann zudem klar, dass die im Text beschriebenen Ausführungsbeispiele nur eine sehr kleine Auswahl in Bezug auf die tatsächlich realisierbaren Möglichkeiten darstellen.

Eine Alternative zu der ,in vitro'-Amidierung mittels PAM ergibt sich, wenn man das Enzym mit dem zu amidierenden Vorläuferprotein in ein und derselben Wirtszelle co-exprimiert. Dies erreicht man, indem man eine Gensequenz, die für eine PAM-Aktivität kodiert unter Kontrolle einer wirtsspezifischen Regulationssequenz in die Wirtszelle einbringt. Diese Expressionssequenz kann entweder stabil in die jeweilige chromosomale DNA Sequenz eingebaut sein, oder auf einem zweiten Plasmid parallel zu dem Expressionsplasmid für das Zielprotein vorliegen, oder als zweite Expressionskassette auf ein und demselben Vektor integriert sein, oder sogar in einem polycistronischen Expressionsansatz in Phase mit der Gensequenz, die das Zielprotein kodiert unter Kontrolle der gleichen Promotor Sequenz kloniert sein.

Die vorliegende Erfindung umfasst somit biotechnologische Verfahren zur Herstellung von Peptiden der Formel I oder deren Derivate, die mindestens 60%, vorzugsweise mindestens 80%, insbesondere mindestens 90% Homologie zur Formel I aufweisen.

Die erfindungsgemäßen Verfahren sind dadurch gekennzeichnet, dass rekombinante Organismen hergestellt werden, die einen Peptidvorläufer synthetisieren, der anschließend in Gegenwart eines Enzyms direkt oder durch Verknüpfung mit einer oder mehreren basischen Aminosäure oder deren Derivaten in Reihe, vorzugsweise Lysin-, Histidin- und/oder Arginin-Resten in Reihe, insbesondere Lysin, wobei die Sequenz C-terminal amidiert ist, in ein Peptid entsprechend der Formel I umgewandelt werden kann.

Weitere Erfindungsgegenstände sind auch die Verwendungen der erfindungsgemäßen Verbindungen der allgemeinen Formel I oder der C-terminal amidierten Peptide der allgemeinen Formel II, die nach dem erfindungsgemäßen Verfahren hergestellt wurden, insbesondere der Verbindungen gemäß Seq ID No 1 oder 2, zur Herstellung eines pharmazeutischen Produktes oder einer pharmazeutischen Formulierung, vorzugsweise zur Behandlung von Kohlenhydratstoffwechsel-Störungen, besonders bevorzugt zur Behandlung von Diabetes mellitus.

### Beispiele

### Beispiel 1. Synthese einer E.coli spezifischen DNA Sequenz kodierend für AVE ₁₋₄₄-Gly

Zunächst wurde die Gensequenz Seq ID No. 3 kodierend für das Peptid AVE ₁₋₄₄-Gly (Seq ID No. 2) hergestellt:

Die Synthese der Gensequenz erfolgte mittels PCR-Technologie. Dazu wurden die folgenden 5 Primer mittels chemischer DNA-Synthese hergestellt. Diese Synthese erfolgte mittels des Expedite^{™} DNA-Synthese-Systems.
a) Primer zp5u hat die Sequenz (Seq ID No 4):
   5'- TTTTTTAAGC TTGCACGGTG AAG -3'
   Seq ID No 4 umfasst den Bereich 1-23 des Sinn-Stranges (,sense').
b) Primer zp3a hat die Sequenz (Seq ID No 5): Seq ID No 5 umfasst den Bereich 1-59 des Gegenstranges (,antisense')
c) Primer zp3b hat die Sequenz (Seq ID No 6): Seq ID No 6 umfasst den Bereich 40-108 des Gegenstranges (,antisense').
d) Primer zp3c hat die Sequenz (Seq ID No 7): Seq ID No 7 umfasst den Bereich 91-164 des Gegenstranges (,antisense').
e) Primer zp3d hat die Sequenz (Seq ID No 8): Seq ID No 8 umfasst den Bereich 14.4.-197 des Gegenstranges (,antisense').

Mit den Primern wurden in Folge 4 PCR-Reaktionen unter Standard-Bedingungen bei 54°C durchgeführt. In Reaktion 1 wurden je 100ng der Primer zp3a und zp5u eingesetzt. Die PCR -Zyklenzahl war 5. In der zweiten Reaktion wurde 1/40 der Reaktion mit je 100ng der Primer zp5u und zp3b in 10 Zyklen umgesetzt. In Reaktion 3 wurden in weiteren 10 Zyklen 1/40 des Produktes der Reaktion 2 mit je 100ng der Primer zp5u und zp3c umgesetzt. Schließlich wurde in 25 PCR Zyklen mit 1/40 der Ausbeute aus Reaktion 3 und den Primern zp5u und zp3d das gewünschte DNA-Fragment synthetisiert, dessen Länge gelelektrophoretisch kontrolliert wurde. Das gewünschte DNA-Fragment wurde gereinigt und mit den Restriktionsenzymen EcoR1 und anschließend mit Hind3 nach Angaben des Herstellers (New England Biolabs) umgesetzt.

Parallel wurde DNA des Plasmides pUC19 (New England Biolabs) mit den Enzymen EcoR1 und Hind3 umgesetzt. Die Fragmente der Spaltansätze wurden über ein 1,2%-iges Agarosegel separiert und anschließend das Restvektorfragment aus pUC19 und das gewünschte Produkt aus Reaktion 4 isoliert. Die gereinigten Fragmente wurden in einer T4-Ligase-Reaktion miteinander über Nacht bei 16°C ligiert. Anschließend wurden kompetente E.coli Zellen (Stratagene, Stamm E. coli XL10 Gold), mit dem Ligationsansatz transformiert und auf Agarplatten enthaltend 25mg/l Ampicillin ausplattiert. Aus den Einzelklonen wurde Plasmid-DNA isoliert und mittels DNA Sequenzanalyse charakterisiert.

Die Plasmid-DNA des gewünschten Fragmentes erhielt die Bezeichnung pSCHPUCZP10. Sie diente als Ausgangsmaterial zur Herstellung von Expressionsvektoren zur Synthese der erfindungsgemäßen Vorläuferpeptide in E.coli K12 Zellen.

### Beispiel 2: Konstruktion von Expressionsvektoren, die das Vorläuferpeptid AVE ₁₋₄₄-Gly kodieren

Zur Herstellung des Peptides AVE ₁₋₄₄ - Gly wurde die kodierende Sequenz in den Vektor pThioHisA der Firma Invitrogen (Catalog No. K360-01) eingeführt. Es entstand ein Fusionsprotein aus Thioredoxin, das über die Enterokinase-Erkennungssequenz DDDDK mit dem Vorläuferpeptid AVE ₁₋₄₄ - Gly verbunden ist. Durch Umsetzung mittels Enterokinase (Invitrogen) wurde AVE ₁₋₄₄-Gly freigesetzt und kann anschließend gemäß Beispiel 7 (unten) in Gegenwart von PAM (Wako Pure Chemicals Ind., Ltd) in das Zielprotein AVE ₁₋₄₄ - NH₂ umgewandelt werden.

### Zwei Primer mit folgender Sequenz werden synthetisiert:

Primer Zp_thiohisf mit einer BamH1-Schnittstelle (Seq ID No 9):
5'- TTTTTTGGAT CCGGTGATGA CGATGACAAG CACGGTGAAG GTACCTTC-3'

### Primer ZP_thiohisrev mit einer EcoR1-Schnittstette (Seq ID No 10):

5' - TTTTTTGAAT TCGTCGACCA GGTGC -3'

In einer PCR-Reaktion mit pSCHPUCZP10 DNA als 'Template' wurden die Primer Zp_thiohisf und ZP_thiohisrev unter Standardkonditionen eingesetzt. Es entstand ein PCR-Fragment, das nach Spaltung mit den Enzymen BamH1 und EcoR1 direkt in den - entsprechend mit BamH1 und EcoR1 geöffneten - pTHtOHisA-Vektor in einer T4-Ligasereaktion eingesetzt wird. Kompetente E. coli BL21 Zellen wurden mit dem Ligationsgemisch transformiert und auf selektivem Agar ausplattiert, der 25mg/l Ampicillin enthielt. Von einigen Klonen wurde die Plasmid-DNA re-isoliert und mittels PCR und anschließender DNA-Sequenzanalyse analysiert. Die gewünschten positiven Klone, die pTHIOHisAZP10-Gly genannt wurden, wurden in analoger Weise gemäß Beispiel 14 des Patents US5496924 auf Expression des Fusionsproteins überprüft. Aufgrund der positiven Expressionsanalyse wurde ein Klon ausgewählt und zur Herstellung von größeren Materialmengen fermentiert. Das entstehende Fusionsprotein umfasst Thioredoxin, das über eine Enterokinaseerkennungs-Sequenz mit AVE ₁₋₄₄-Gly verbunden ist (Seq ID No 12).

US5496924 schlägt ein Expressionssystem vor, das grundsätzlich die Herstellung von maßgeschneiderten Fusionsproteinen erlaubt. Der Vorteil des Systems liegt darin, dass man Fusionsproteine mit kleinem Ballastanteil herstellen kann. Fusioniert man die Sequenzabschnitte A-B über die Enterokinase-Erkennungssequenz DDDDK mit AVE ₁₋₄₄ - Gly, so erhält man ein Fusionsprotein, mit folgender Gen- und Aminosäuresequenz (Seq ID No. 11 und 12):

Die Herstellung der kodierenden Gensequenz erfolgte mittels PCR-Technologie. Dazu wurden folgende Primer synthetisiert:
1) Primer psw3_zpcolf (Seq ID No 13):
   5'- CGTATCGACG ATGACGATAA ACACGGTGAA GGTACCTTC -3'
   Die Sequenz des Primers überdeckt dabei die Enterokinaseerkennungstelle und den Anfang der AVE ₁₋₄₄ - Gly kodierenden Sequenz.
2) Primer psw3_zpcolrev (Seq ID No 14):
   5'- GTGTTTATCG TCATCGTCGA TACGCGTCAG TTTCGG -3'
   Die Sequenz entspricht dabei der synthetischen interleukin2-Sequenz, die entsprechend Tabelle I von US5496924 die Aminosäuren 34 -38 sowie 2/3 des Codons für die Aminosäure Methionin überdeckt. Der Rest der Primersequenz überlappt mit Primer psw3_zpcolf.
3) pBprimef1 (Seq ID No 15):
   5' - TGAGCGGATA ACAATTTCAC AC -3'
   Der Primer hybridisiert stromaufwärts mit der EcoRI-Schnittstelle, die in Plasmid pK50 (Figur 33 der US5496924) enthalten ist.
4) psw3_zp10colrev mit Hind3 Schnittstelle (Seq ID No 16):
   5' - TTTTTTAAGC TTGCGGCCGC ACGTGCATGC TATTATCAAC CTTC- 3'
   Zwei PCR's wurden parallel durchgeführt. Die eine wurde auf DNA des Plasmides pK50 mit dem Primerpaar pBprimef1 und psw3_zpcolrev bei 50°C und die andere Reaktion mit dem Primerpaar psw3_zpcolf und psw3_zp10colrev bei 54°C auf DNA des Plasmides pTHIOHisAZP10-Gly durchgeführt. Die PCR Ausbeuten wurden nach gelelektrophoretischer Auftrennung gereingt, je ein Aliquot im Verhältnis 1:1 gemischt und anschließend in einer dritten PCR mit dem Primerpaar pBprimef1 und psw3_zp10colrev umgesetzt. Die PCR Ausbeute wurde mit den Enzymen EcoR1 und Hind3 umgesetzt und in das parallel mit diesen Enzymen geöffnete Plasmid pK50 in einer T4-Ligasereaktion eingesetzt. Kompetente E.coli BL21 Zellen werden mit dem Ligationsgemisch transformiert und auf selektivem Agar, der 25mg/l Ampicillin enthielt, ausplattiert. Von einigen Klonen wurde Plasmid DNA re-isoliert und mittels PCR und anschließender DNA-Sequenzanalyse analysiert. Positive Klone wurden pBZP100 genannt, und auf Expression des Fusionsproteins überprüft.
   Die Expressionsprodukte wurden massenspektrometrisch und mittels SDS-PAGE analysiert und der N-Terminus mittels Proteinsequenzanalyse bestimmt. Ein geeigneter Klon zur Fermentierung größerer Materialmengen wurde ausgewählt.

### Beispiel 3: Fermentation der in Beispiel 2 konstruierten Stämme

E. coli BL21-Zellen, transformiert mit verschiedenen, für Zielpeptid-Derivate(-Fusionsprotein) kodierenden Plasmid-Vektoren, wurden in Mineralsalzmedium oder Komplexmedium (siehe Beispiel 1) bei 30°C oder 37°C und einem pH-Wert von 7,0 in einem Fermenter kultiviert. Die pH-Einstellung erfolgte mit einer NH₄⁺-Lösung (26% in Wasser). Die Belüftung der Kultur wurde durch eine Regelungsstrategie, die den Gelöstsauerstoff in der Kulturbrühe konstant bei 30% hielt, sichergestellt. Für Fed Batch Prozesse in Mineralsalzmedium wurde nach Beendigung der Batch-Phase eine Glucoselösung (60% w/v) zugefüttert (8 g/L/h bis 26g/L/h). Die Induktion der Proteinexpression erfolgte durch Zugabe von IPTG (1 - 4 mM Endkonz. (f.c.)). Die Zeitdauer der Induktion betrug 6-8 h. Die Expression der Zielproteine wurde durch SDS-Polyacrylamid-Gelelektrophorese (SDS-PAGE) nachgewiesen.

Die Expression von AVE₁₋₄₄-Gly(-Fusionsprotein) in E. coli BL21/pBZP100 wurde wie nachfolgend beschrieben durchgeführt:
Aus einer bei -80°C gelagerten Dauerkultur von E. coli BL21-Zellen wurden 100 µL Zellsuspension entnommen und in 0,5 L Vorkulturmedium bei 37°C schüttelnd für 10-16 h inkubiert. Die Hauptkultur im Fermenter wurde auf eine Animpfdichte von 0,01 bis 0,05 OD₆₀₀ mit der entsprechenden Menge Vorkultur inokuliert.

Vorkulturmedium:
5 g/L Bacto trypton
10 g/L Hefeextrakt
5 g/L NaCl

Hauptkulturmedium:
Definiertes Mineralsalzmedium (Minimalmedium) basierend auf Glukose als Kohlenstoffquelle (Jeffrey H. Miller: Experiments in Molecular Genetics, Cold Spring Harbor Laboratory (1972)).

Nach Aufbrauchen der initial im Hauptkulturmedium vorhandenen Glucose wurde eine Glucoselösung zugefüttert. Die Proteinexpression durch Zugabe von IPTG (1 mM f.c.) induziert und die maximale Expression des Fusionsproteins nach der Induktion beobachtet.

Unter Verwendung z.B. des SDS-PAGE Analysensystems der Firma Novex (NuPage^{®} Novex 12% Gelsystem, Invitrogen^{™}) wurden entsprechend der Angaben des Herstellers in der Fermentation je 0,02 OD₆₀₀ₙₘ Zellsuspension, die zu unterschiedlichen Kultivierungszeitpunkten dem Fermenter entnommen wurden, analysiert.

### Beispiel 4: Reinigung des Fusionsproteins

Isolierung des BZP-AVE₁₋₄₄-Gly-Fusionsproteins:
200 g Biomasse eines rekombinanten E. coli-Stammes wurden in 300 ml Tris-Puffer (50 mM Tris/HCl, pH 7,4; 1 mM EDTA) resuspendiert. Der Zellaufschluss erfolgte durch zweimalige Hochdruckhomogenisation (Rannie-Hochdruckhomogenisator, 1000 bar). Unlösliche Bestandteile im Homogenisat wurden durch Zentrifugation abgetrennt. Der Überstand wurde druckfiltriert (Sartorius 0,22 µm Filter, Typ111) und auf eine zuvor mit Puffer (50 mM Tris/HCl pH 7,3; 1 mM EDTA) äquilibrierte Chromatographiesäule (Source S, Amersham Biosciences) aufgetragen. Nach dem Probenauftrag erfolgte ein Waschschritt mit Äqulibrierungspuffer (2 Säulenvolumen), gefolgt von einem weiteren Waschschritt mit 10% Hochsalzpuffer (50 mM Tris/HCl pH 7,3; 1 M NaCl, 1 mM EDTA). Die Fraktionierung erfolgte durch Anlegen eines Salzgradienten mittels Hochsalzpuffer über 5 Säulenvolumen. Die Überprüfung des Fusionsproteingehalts der einzelnen Fraktionen erfolgte über SDS-Gelelektrophorese (NuPage^{®} Novex 12% Gelsystem, Invitrogen). Fusionsprotein-haltige Fraktionen wurden vereinigt und zwischen 5- und 10-fach ankonzentriert (Millipore Ultrafiltrationszelle, 10 kDa cut-off Membran). Das Konzentrat wurde nach Puffertausch in Enterokinasepuffer (50 mM Tris/HCl pH 7,4; 50 mM NaCl, 2mM CaCl₂) direkt zur Proteasespaltungsreaktion eingesetzt, oder vor der Spaltreaktion über Gelfiltration (Superdex 75, Amersham Biosciences) zusätzlich gereinigt.

Die Spaltung der Fusionsproteine erfolgte mittels Enterokinase (Invitrogen) in Enterokinase-Puffer (20 mM Tris/HCl, 50 mM NaCl, 2 mM CaCl₂ pH 7,4) nach Angaben des Herstellers.

### Beispiel 5: Aufreinigung eines Thioredoxin-Fusionsproteins enthaltend Seq ID No. 3

200 g Biomasse eines rekombinanten E. coli-Stammes wurden in 50 mM Tris-Puffer (pH 7,4; 1 mM EDTA) resuspendiert. Der Zellaufschluss erfolgte durch zweimalige Hochdruckhomogenisation (Rannie-Hochdruckhomogenisator, 1000 bar). Unlösliche Bestandteile im Homogenisat wurden durch Zentrifugation abgetrennt. Der Überstand wurde druckfiltriert (Sartorius 0,22 µm Filter, Typ111) und auf eine zuvor mit Puffer (50 mM Tris/HCl pH 7,4; 1 mM EDTA) äquilibrierte Chromatographiesäule (Source Q, Amersham Biosciences) aufgetragen. Nachdem Probenauftrag erfolgte ein Waschschritt mit Äquilibrierungspuffer (2 Säulenvolumen) und die Fraktionierung erfolgte durch Anlegen eines Salzgradienten mittels Hochsalzpuffer (50 mM Tris/HCl pH 7,4; 0,3 M NaCl, 1' mM EDTA) über 6 Säulenvolumen. Die Überprüfung des Fusionsproteingehalts der einzelnen Fraktionen erfolgte über SDS-Gelelektrophorese ((NuPage^{®} Novex 12% Gelsystem, Invitrogen^{™}). Fusionsprotein-haltige Fraktionen wurden vereinigt und 5-10-fach ankonzentriert (Millipore Ultrafiltrationszelle, 10 kDa cut-off Membran). Das Konzentrat wurde durch Gelfiltrationschromatographie (Superdex 75, Amersham Biosciences) weiter fraktioniert. Eine zuvor äquilibrierte Säule (50 mM Tris/HCl pH 7,4; 200 mM NaCl) wurde mit bis zu 5% des Säulenvolumes mit ankonzentrierter Fusionsproteinlösung beladen. Die Elution erfolgt durch Spülen mit Äquilibrierungspuffer. Die Überprüfung des Fusionsproteingehalts der einzelnen Fraktionen erfolgte erneut über SDS-Gelelektrophorese ((NuPage^{®} Novex 12% Gelsystem, Invitrogen^{™}. Die entsprechenden Fraktionen wurden vereinigt, auf ca. 5 mg/ml ankonzentriert (Vivaspin-Konzentratoren mit 10 kD cut- off, Vivascience) und mittels Diafiltrationseinsätzen (Vivascience) erfolgte der Puffertausch in Enterokinasepuffer (20 mM Tris/HCl pH 7,4; 50 mM NaCl).

Die Prozessierung der AVE₁₋₄₄-Gly-Vorstufe erfolgte anschließend mittels Enterokinase analog zu Beispiel 4.

### Beispiel 6: Trennung der Spaltprodukte aus der Enterokinase-Spaltreaktion

Nach der Spaltung der Fusionsproteine mittels Enterokinase wurden die Spaltprodukte voneinander durch Ionenaustauschchromatographie (Source 30S, Amersham Biosciences) getrennt. Die Ionenstärke der Lösung wurde auf ca. 10 mS/cm durch Zugabe von Natriumchlorid gebracht. Nach Aufgabe der Proteinlösung auf die zuvor äquilibrierte Säule (20 mM Tris/HCl, pH 7.4; mit NaCl auf eine Leitfähigkeit von ca. 10 mS/cm eingestellt) wurde nicht gebundenes Material mit Puffer (20 mM Tris/HCl, pH 7.4; mit NaCl auf eine Leitfähigkeit von ca. 10 mS/cm eingestellt) herausgewaschen. Die Elution des Ave₁₋₄₄-Gly Peptids erfolgte durch Anlegen eines Gradienten über 10 Säulenvolumen auf 500 mM NaCl.

Die Identifizierung AVE₁₋₄₄-haltiger Fraktionen oder Vorstufen zu AVE₁₋₄₄ erfolgte durch SDS-Gelelektrophorese , HPLC und massenspektrometrisch. Die entsprechenden Fraktionen wurden vereinigt und nach Entfernen von organischem Lösungsmittel lyophilisiert.

Das isolierte AVE₁₋₄₄-Gly wurde zur Absicherung der Aminosäuresequenz abschließend via Edman vollständig sequenziert.

### Beispiel 7: Umwandlung von AVE₁₋₄₄- Gly zu AVE₁₋₄₄ -NH₂

Die Reaktion wurde mit Hilfe des Enzyms PAM (Peptidiylglycine-amidating enzyme Wako Pure Chemicals Ind., Ltd. ( Best. Nr. 161-16971)) nach Angaben zu den Reaktionsbedingungen des Herstellers durchgeführt.

Folgende Lösung wurde angesetzt:
1 µM CuSO₄
5 mMKI
3 mM Na-Ascorbat
230 U/ml Katalase (Rind, Fluka)
600 U/ml PAM (Wako Chemicals)
0.1 M Tris/HCl pH 7.0 mit 0.001 % Triton X-100

Die Lösung wurde bei 37°C 1 h vorinkubiert und dann die Proteinlösung AVE₁₋₄₄Gly (Tris/HCl pH 7.0, 80 µg/ml Endkonzentration) zugegeben. Die Reaktionsmischung wurde dann bei 37° C weiter inkubiert. Der Reaktionsverlauf wird durch Probennahme zu verschiedenen Zeiten verfolgt. Bei maximaler Umsetzung wurde die Reaktion durch Zugabe einer 50mM EDTA-Lösüng gestoppt

Das Reaktionsgemisch wurde anschließend über lonentauscherchromatographie (Fa. Shodex, Säule Typ IEC CM- 825 (8x75 mm)) aufgetrennt. An diese Säule wurde folgender Gradient angelegt:
Eluent A - 40 mMol Phosphatpuffer pH 7 + 20% Acetonitril
Eluent B-50 mMol Phosphatpuffer pH 7 + 1 M NaCL

Die Säule wurde bei Raumtemperatur mit einer Durchflussrate von 2ml/min oder 1 ml/min betrieben.

Die eluierten Fraktionen (Detektion bei 280nm) wurden aufgefangen und die Masse des jeweiligen Peptides über MALDI-MS bestimmt. Die massenspektrometrischen Analysen wurden mit einem Gerät des Typs BRUKER Reflex IV durchgeführt. Die Proben wurden direkt für die MALDI-MS-Analytik eingesetzt, bzw. auf eine Konzentration von ca. 50 pmol/µL mit 50% TAaq ([1+1] 0,1% TFA + 50% Acetonitril) verdünnt.

Die erwartete Masse für das AVEI₁₋₄₄ - NH₂ wurde bestätigt. Das erhaltene Produkt kann der weiteren pharmazeutischen Verwendung zugeführt werden.

Pharmazeutische Formulierungen können im weiteren durch Beigabe geeigneter pharmazeutischer Formulierungshilfsstoffe zu dem biologisch aktiven Peptid bzw. Peptid-Derivat in einer dem Fachmann bekannten Weise hergestellt werden.

## Patentansprüche

1. Verfahren zur Herstellung von C-terminal amidierten Peptiden der allgemeinen Formel II
(AS)n-Xm-NH₂ (Formel II),
worin
AS eine oder mehrere genetisch codierbare Aminosäure bedeutet;
n 5-2000 ist, vorzugsweise 10-1000, insbesondere 15-500, ganz besonders bevorzugt 20-400;
und (AS)n und/oder (AS)nXm vorzugsweise ein biologisch aktives Peptid bzw. Protein bedeutet;
X ein oder mehrere basische Aminosäure oder deren Derivate, vorzugsweise Lysin, Histidin und/oder Arginin, insbesondere Lysin bedeutet;
m 1-15 ist, vorzugsweise 3-10, insbesondere 6-8;
n und m ganze Zahlen bedeuten, und
worin man
a) Wirtszellen in einem geeigneten Nährmedium kultiviert,
b) eine Verbindung der allgemeinen Formel I
(AS)n-XmYp (Formel I),
worin Y eine oder mehrere ladungsneutrale Aminosäure bedeutet, vorzugsweise Glycin, p 1-10 ist, vorzugsweise 1-5, insbesondere 1; bedeutet, exprimiert,
c) gegebenenfalls die Verbindung der Formel I aus einem geeigneten Vorläuferpeptid mittels enzymatischer oder chemischer Spaltung freisetzt;
d) die Expressionsprodukte aus Schritt b) bzw. die Zwischenprodukte aus Schritt c)-gegebenenfalls nach einem Reinigungsschritt-mit einem alpha-amidierenden Enzym (PAM) umsetzt; und
e) das C-terminal amidierte Peptid der allgemeinen Formel II in geeigneter Weise aufreinigt, vorzugsweise durch ein präparatives chromatographisches Verfahren.

2. Verfahren gemäß Anspruch 1 bei dem die Verbindung der Formel I durch SEQ ID No. 2 definiert ist, sowie deren biologisch aktive Derivate mit einer Homologie von mindestens 60 %.

3. Verfahren nach einem oder mehreren der Ansprüche 1-2, worin die Verbindung der Formel I aus geeigneten Vorläuferpeptiden mittels enzymatischer Spaltung freigesetzt wird.

4. Verfahren nach Anspruch 3, worin die Verbindung der Formel I aus geeigneten Vorläuferpeptiden mittels des Enzyms Enterokinase freigesetzt wird.

5. Verfahren gemäß Anspruch 1 zur Herstellung einer Verbindung der Formel II, die durch die Sequenz SEQ ID NO. 1 definiert ist.

## Claims

1. A method for the production of C-terminally amidated peptides of the general formula II
(AS)n-Xm-NH₂ (Formel II),
wherein
AS is one or more genetically encodable amino acids;
n is 5-2000, preferably 10-1000, in particular 15-500, quite especially preferably 20-400;
and (AS)n and/or (AS) nXm preferably is a biologically active peptide or protein;
X is one or more basic amino acids or derivatives thereof, preferably lysine, histidine and/or arginine, in particular lysine;
m is 1-15, preferably 3-10, in particular 6-8; and
n and m are whole numbers and
wherein
a) host cells are cultured in a suitable nutrient medium,
b) a compound of the general formula I
(AS)n-XmYp (formula I)
wherein Y is one or more neutral charge amino acids, preferably glycine, p is 1-10, preferably 1-5, in particular 1; is expressed,
c) optionally the compound of the formula I is released from a suitable precursor peptide by enzymatic or chemical cleavage;
d) the expression products from step b) or the intermediate products from step c) - optionally after a purification step - are reacted with an alpha-amidating enzyme (PAM); and
e) the C-terminal amidated peptide of the general formula II is purified in a suitable manner, preferably by a preparative chromatographic method.

2. A method as claimed in claim 1, in which the compound of the formula I is defined by SEQ ID No.2, and biologically active derivatives thereof with a homology of at least 60%.

3. A method as claimed in one or more of claims 1-2, wherein the compound of the formula I is released from suitable precursor peptides by enzymatic cleavage.

4. A method as claimed in claim 3, wherein the compound of the formula I is released from suitable precursor peptides by means of the enzyme enterokinase.

5. A method as claimed in claim 1 for the production of a compound of the formula II which is defined by the sequence SEQ ID NO. 1.

## Revendications

1. Procédé pour la préparation de peptides amidés en l'extrémité C de formule générale II
(AS)ₙ-Xₘ-NH₂ (Formule II),
où
AS signifie un ou plusieurs acides aminés pouvant être codés génétiquement ;
n vaut 5-2000, de préférence 10-1000, en particulier 15-500, de manière tout particulièrement préférée 20-400 ;
et (AS)ₙ et/ou (AS)ₙXₘ signifie(nt) de préférence un peptide biologiquement actif ou une protéine biologiquement active ;
X signifie un ou plusieurs acides aminés basiques ou leurs dérivés, de préférence lysine, histidine et/ou arginine, en particulier lysine ;
m vaut 1-15, de préférence 3-10, en particulier 6-8 ;
n et m signifient des nombres entiers, et
dans lequel
a) on cultive des cellules hôtes dans un milieu nutritif approprié,
b) on exprime un composé de formule générale I
(AS)ₙ-XₘYₚ (formule I),
dans laquelle Y signifie un ou plusieurs acides aminés à charge neutre, de préférence glycine, p vaut 1-10, de préférence 1-5, en particulier 1 ;
c) on libère le cas échéant le composé de formule I à partir d'un peptide précurseur approprié par un clivage enzymatique ou chimique ;
d) on transforme les produits d'expression de l'étape b) ou les produits intermédiaires de l'étape c) - le cas échéant après une étape de purification - avec une enzyme qui amide en position alpha (PAM) ; et
e) on purifie le peptide amidé en l'extrémité C de formule générale II de manière appropriée, de préférence par un procédé de chromatographie préparative.

2. Procédé selon la revendication 1 dans lequel le composé de formule I est défini par la SEQ ID No. 2, ainsi que par ses dérivés biologiquement actifs présentant une homologie d'au moins 60%.

3. Procédé selon l'une ou plusieurs des revendications 1-2, dans lequel le composé de formule I est libéré à partir de peptides précurseurs appropriés au moyen d'un clivage enzymatique.

4. Procédé selon la revendication 3, dans lequel le composé de formule I est libéré à partir de peptides précurseurs appropriés au moyen de l'enzyme entérokinase.

5. Procédé selon la revendication 1 pour la préparation d'un composé de formule II, qui est défini par la séquence SEQ ID NO. 1.


### SEQUENZPROTOKOLL

<110> Aventis Pharma Deutschland GmbH
<120> Verfahren zur Herstellung von Carboxy terminal amidierten Peptiden mit GLP1 Wirkung
<130> DEAV2004/0076
<140>
   <141>
<160> 16
<170> PatentIn Ver. 2.1
<210> 1
   <211> 44
   <212> PRT
   <213> Künstliche Sequenz
<220>
   <223> Beschreibung der künstlichen sequenz:synthetisches Peptid;MOD_RES K44 Amidation
<400> 1
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Ser Lys Lys Lys Lys Lys Lys
           35                  40
<210> 2
   <211> 45
   <212> PRT
   <213> Künstliche Sequenz
<220>
   <223> Beschreibung der künstlichen sequenz:synthetisches Peptid
<400> 2
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Ser Lys Lys Lys Lys Lys Lys Gly
           35                  40                  45
<210> 3
   <211> 198
   <212> DNA
   <213> Künstliche Sequenz
<220>
   <223> Beschreibung der künstlichen Sequenz:synthetische DNA
<400> 3
   ttttttaagc ttgcacggtg aaggtacctt cacctccgac ctgtccaaac agatggaaga 60
   agaagctgtt cgtctgttca tcgaatggct gaaaaacggt ggtccgtcct ccggtgctcc 120
   gccttcgaaa aagaagaaaa agaaaggttg ataatagcat gcacgtgcgg ccgcacctgg 180
   tcgacgaatt caaaaaaa                                                198
<210> 4
   <211> 23
   <212> DNA
   <213> Künstliche Sequenz
<220>
   <223> Beschreibung der künstlichen sequenz:Primer
<400> 4
   ttttttaagc ttgcacggtg aag 23
<210> 5
   <211> 59
   <212> DNA
   <213> Künstliche Sequenz
<220>
   <223> Beschreibung der künstlichen sequenz:Primer
<400> 5
   cttccatctg tttggacagg tcggaggtga aggtaccttc accgtgcaag cttaaaaaa 59
<210> 6
   <211> 69
   <212> DNA
   <213> Künstliche Sequenz
<220>
   <223> Beschreibung der künstlichen Sequenz:Primer
<400> 6
   ggacggacca ccgtttttca gccattcgat gaacagacga acagcttctt cttccatctg 60
   tttggacag 69
<210> 7
   <211> 74
   <212> DNA
   <213> Künstliche Sequenz
<220>
   <223> Beschreibung der künstlichen Sequenz:Primer
<400> 7
   gtgcatgcta ttatcaacct ttctttttct tctttttcga aggcggagca ccggaggacg 60
   gaccaccgtt tttc 74
<210> 8
   <211> 54
   <212> DNA
   <213> Künstliche Sequenz
<220>
   <223> Beschreibung der künstlichen Sequenz:Primer
<400> 8
   ttttttgaat tcgtcgacca ggtgcggccg cacgtgcatg ctattatcaa cctt 54
<210> 9
   <211> 48
   <212> DNA
   <213> Künstliche Sequenz
<220>
   <223> Beschreibung der künstlichen Sequenz:Primer
<400> 9
   ttttttggat ccggtgatga cgatgacaag cacggtgaag gtaccttc 48
<210> 10
   <211> 25
   <212> DNA
   <213> Künstliche Sequenz
<220>
   <223> Beschreibung der künstlichen Sequenz:Primer
<400> 10
   ttttttgaat tcgtcgacca ggtgc 25
<210> 11
   <211> 321
   <212> DNA
   <213> Künstliche Sequenz
<220>
   <223> Beschreibung der künstlichen Sequenz:synthetische DNA
<400> 11
   ggaaacagaa ttcatggcgc cgacctcttc ttctaccaaa aagactcaac tgcaactgga 60
   acacctgctg ctggacctgc agatgatcct gaacggtatc aacaactaca aaaacccgaa 120
   actgacgcgt atcgacgatg acgataaaca cggtgaaggt accttcacct ccgacctgtc 180
   caaacagatg gaagaagaag ctgttcgtct gttcatcgaa tggctgaaaa acggtggtcc 240
   gtcctccggt gctccgcctt cgaaaaagaa gaaaaagaaa ggttgataat agcatgcacg 300
   tgcggccgca agcttaaaaa a                                           321
<210> 12
   <211> 90
   <212> PRT
   <213> Künstliche Sequenz
<220>
   <223> Beschreibung der künstlichen Sequenz:synthetisches Peptid
<400> 12
   Met Ala Pro Thr Ser Ser Ser Thr Lys Lys Thr Gln Leu Gln Leu Glu
   1               5                   10                  15
   His Leu Leu Leu Asp Leu Gln Met Ile Leu Asn Gly Ile Asn Asn Tyr
               20                  25                  30
   Lys Asn Pro Lys Leu Thr Arg Ile Asp Asp Asp Asp Lys His Gly Glu
           35                  40                  45
   Gly Thr Phe Thr Ser Asp Leu Ser Lys Gln Met Glu Glu Glu Ala Val
       50                  55                  60
   Arg Leu Phe Ile Glu Trp Leu Lys Asn Gly Gly Pro Ser Ser Gly Ala
   65                  70                  75                  80
   Pro Pro Ser Lys Lys Lys Lys Lys Lys Gly
                   85                  90
<210> 13
   <211> 39
   <212> DNA
   <213> Künstliche Sequenz
<220>
   <223> Beschreibung der künstlichen Sequenz:Primer
<400> 13
   cgtatcgacg atgacgataa acacggtgaa ggtaccttc 39
<210> 14
   <211> 36
   <212> DNA
   <213> Künstliche Sequenz
<220>
   <223> Beschreibung der künstlichen Sequenz:Primer
<400> 14
   gtgtttatcg tcatcgtcga tacgcgtcag tttcgg 36
<210> 15
   <211> 22
   <212> DNA
   <213> Künstliche Sequenz
<220>
   <223> Beschreibung der künstlichen Sequenz:Primer
<400> 15
   tgagcggata acaatttcac ac 22
<210> 16
   <211> 44
   <212> DNA
   <213> Künstliche Sequenz
<220>
   <223> Beschreibung der künstlichen Sequenz:Primer
<400> 16
   ttttttaagc ttgcggccgc acgtgcatgc tattatcaac cttc 44